# EUROPÄISCHE PATENTSCHRIFT

(11) **EP 1 092 086 B1**
(45) Veröffentlichungstag und Bekanntmachung des Hinweises auf die Patenterteilung: **12.03.2003**
(21) Anmeldenummer: 99936388.0
(22) Anmeldetag: 01.06.1999
(51) Int. Cl.: F02D 41/14, F01N 11/00

(54) **VERFAHREN ZUR ÜBERPRÜFUNG DES DYNAMIKVERHALTENS EINES MESSAUFNEHMERS IM ABGASTRAKT EINER BRENNKRAFTMASCHINE**
METHOD FOR TESTING THE DYNAMIC BEHAVIOR OF A MEASURE SENSOR IN THE EXHAUST SYSTEM OF AN INTERNAL COMBUSTION ENGINE
PROCEDE DE CONTROLE DU COMPORTEMENT DYNAMIQUE D'UN CAPTEUR DE MESURE MONTE DANS LE CIRCUIT D'ECHAPPEMENT D'UN MOTEUR A COMBUSTION INTERNE

(30) Priorität: 29.06.1998 DE 19828929
(43) Veröffentlichungstag der Anmeldung: 18.04.2001
(73) Patentinhaber: SIEMENS AKTIENGESELLSCHAFT, 80333 München (DE)
(72) Erfinder: ZHANG, Hong, D-93057 Regensburg (DE); RÖSSLER, Jürgen, D-93053 Regensburg (DE); PFLEGER, Corinna, D-93093 Donaustauf (DE)
(86) Internationale Anmeldenummer: DE9901620
(87) Internationale Veröffentlichungsnummer: WO00000728

(56) Entgegenhaltungen:
- EP-A- 0 616 119
- EP-A- 0 637 684
- WO-A-90/09517
- US-A- 5 235 957
- US-A- 5 370 101

## Beschreibung

Die Erfindung betrifft ein Verfahren zur Überprüfung des Dynamikverhaltens eines Meßaufnehmers im Abgastrakt einer mit Luftüberschuß arbeitenden Brennkraftmaschine gemäß dem Oberbegriff des Patentanspruchs 1.

Um den Kraftstoffverbrauch von Otto-Brennkraftmaschinen weiter zu reduzieren, kommen Brennkraftmaschinen mit magerer Verbrennung immer häufiger zum Einsatz. Zur Erfüllung der geforderten Abgasemissionsgrenzwerte ist bei solchen Brennkraftmaschinen eine spezielle Abgasnachbehandlung notwendig. Dazu werden NOx-Speicherkatalysatoren verwendet. Diese NOx-Speicherkatalysatoren sind aufgrund ihrer Beschichtung in der Lage, NOx-Verbindungen aus dem Abgas zu absorbieren, die in einer Speicherphase bei magerer Verbrennung entstehen. Während einer Regenerationsphase werden die absorbierten bzw. gespeicherten NOx-Verbindungen unter Zugabe eines Reduktionsmittels in unschädliche Verbindungen umgewandelt. Als Reduktionsmittel für mager betriebene Otto-Brennkraftmaschinen können CO, H₂ und HC (Kohlenwasserstoffe) verwendet werden. Diese werden durch kurzzeitiges Betreiben der Brennkraftmaschine mit einem fetten Gemisch erzeugt, und dem NOx-Speicherkatalysator als Abgaskomponenten zur Verfügung gestellt, wodurch die gespeicherten NOx-Verbindungen im Katalysator abgebaut werden.

Zur Steuerung der Regenerations- und Speicherphasen bzw. zur Überprüfung des NOx-Speicherkatalysators ist stromab des NOx-Speicherkatalysators im Abgastrakt ein Meßaufnehmer vorgesehen, der mindestens eine Stoffkonzentration erfaßt. Üblicherweise werden Meßaufnehmer verwendet, die ein die NOx-Konzentration darstellendes NOx-Signal abgeben. Ein solcher Meßaufnehmer ist beispielsweise aus der Veröffentlichung "Performance of Thick Film NOx Sensor on Diesel and Gasoline Engines", N. Kato, Y. Hamada und H. Kurachi, Society of Automotive Engineers, Publ.No. 970858 bekannt.

Um die Einhaltung der geforderten Abgasemissionsgrenzwerte über die gesamte Nutzlebensdauer einer solchen Brennkraftmaschine sicherzustellen, wird zunehmend eine Selbstdiagnose (On-Board-Diagnose = OBD) des gesamten Abgasnachbehandlungssystems verlangt. Eine solche OBD ist insbesondere für das Dynamikverhalten eines eingesetzten Meßaufnehmers erforderlich. Dabei soll eine Verlangsamung im Ansprechen des Meßaufnehmers festgestellt und bei ungenügendem Dynamikverhalten ein fehlerhafter Meßaufnehmer diagnostiziert werden können.

Im Zusammenhang mit lambda-geregelten Brennkraftmaschinen, bei denen der Lambda-Wert an einer stromauf eines Katalysators angeordneten Lambda-Sonde eine definierte Schwingung um Lambda = 1 ausführt, ist es bereits bekannt geworden, die Schaltzeiten von Fett nach Mager und von Mager nach Fett zu ermitteln. Dies wird beispielsweise mit einem geeigneten Zeitzähler durchgeführt. Durch getrennte Auswertung der Schaltzeiten von Fett nach Mager und von Mager nach Fett und einen entsprechenden Grenzwertvergleich wird dann die Lambda-Sonde auf einen Defekt überprüft. Dieses Vorgehen ist jedoch für einen stromab eines NOx-Speicherkatalysators angeordneten Meßaufnehmer nicht geeignet, da die Schwingung des Signals um Lambda = 1 durch den NOx-Katalysator gedämpft wird, so dass es dann nicht möglich ist, ein verlangsamtes Ansprechen bzw. ungenügendes Dynamikverhalten des Meßaufnehmers zu erkennen (EP 0 616 119 A).

Es ist somit Aufgabe der vorliegenden Erfindung, ein Verfahren anzugeben, das die Überprüfung des Dynamikverhaltens eines stromab eines NOx-Speicherkatalysators angeordneten, eine Stoffkonzentration erfassenden Meßaufnehmers ermöglicht.

Die Aufgabe wird erfindungsgemäß durch die Merkmale des Anspruchs 1 gelöst.

Die Erfindung geht von der Erkenntnis aus, daß in der Regenerationsphase des NOx-Speicherkatalysators Stoffkonzentrationsänderungen mit vorbestimmten Änderungsraten auftreten. Somit kann durch Überwachung der Änderungsrate einer Stoffkonzentration die Dynamik des Meßaufnehmers überprüft werden. Je nach Ausführungsform des erfindungsgemäßen Verfahrens können dadurch mehrere, unterschiedliche Stoffkonzentrationen darstellende Signale eines Meßaufnehmers auf ihre Dynamik überprüft werden. Insbesondere kommen ein die NOx-Konzentration darstellendes NOx-Signal, ein den Lambda-Wert darstellendes Lambda-Signal bzw. ein die Sauerstoffkonzentration darstellendes O₂-Signal in Frage.

Daß die Änderungsrate einer oder mehrerer Stoffkonzentrationen in der Regenerationsphase überwacht wird, ist deshalb von Bedeutung, da dann die Änderungen der Stoffkonzentration durch Reaktionen im NOx-Speicherkatalysator selbst verursacht werden, bzw. erzeugte Änderungen des Lambda-Wertes mit ausreichender Amplitude am Meßaufnehmer anliegen. An keinem anderen Betriebspunkt kann eine solche Dynamik erzeugt werden, da bei Lambda ≈ 1-Betrieb die Schwankungen in der Stoffkonzentration bzw. des Lambda-Wertes stromab des Katalysators zu gering sind, bzw. vom Katalysator gedämpft werden. Diese Dämpfung macht im normalen Betrieb eine Generierung von Stoffkonzentrationsänderungen mit hoher Änderungsrate zur Überprüfung der Meßaufnehmerdynamik durch Gemischänderungen beim Betrieb der Brennkraftmaschine unmöglich.

Vorteilhafte Ausgestaltungen des erfindungsgemäßen Verfahrens sind in den Unteransprüchen gekennzeichnet.

Die Erfindung wird in einer vorteilhaften Ausführungsform unter Bezugnahme auf die Zeichnung nachstehend näher erläutert. Die Zeichnung zeigt:
- Fig. 1: ein Blockschaltbild des Abgastraktes einer Brennkraftmaschine mit Abgasnachbehandlungssystem,
- Fig. 2: den zeitlichen Verlauf des NOx-Signals eines stromab eines NOx-Speicherkatalysators angeordneten NOx-Meßaufnehmers während einer Regenerationsphase und
- Fig. 3: den zeitlichen Verlauf des Lambda-Signals eines solchen Meßaufnehmers, der zusätzlich zum NOx-Signal auch ein den Lambda-Wert darstellendes Lambda-Signal abgibt.

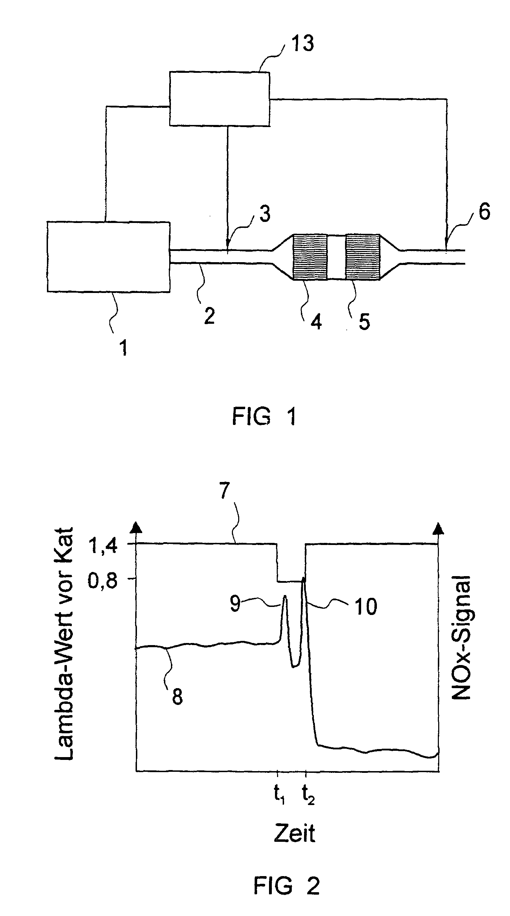

Die in Fig. 1 dargestellte Brennkraftmaschine 1 verfügt in ihrem Abgastrakt 2 über ein Abgasnachbehandlungssystem, das OBD-Fähigkeiten haben soll. Im Abgastrakt 2 der Brennkraftmaschine 1 ist ein Drei-Wege-Vorkatalysator 4 und ein NOx-Speicherkatalysator 5 angeordnet. Es ist auch ein einziger Katalysator möglich, der beide Eigenschaften zeigt. Stromauf dieser beiden Katalysatoren befindet sich eine Vorkat-Lambda-Sonde 3 und stromab davon ein NOx-Meßaufnehmer 6. Der Betrieb der Brennkraftmaschine 1 und des Abgasnachbehandlungssystems wird von einem Betriebssteuergerät 13 gesteuert, das u.a. die Meßwerte der Vorkat-Lambda-Sonde 3 und des NOx-Meßaufnehmers 6 zugeführt bekommt und die OBD durchführt. In dieser OBD soll das Dynamikverhalten des NOx-Meßaufnehmers 6 überprüft werden. Der NOx-Meßaufnehmer 6 gibt neben dem die NOx-Konzentration darstellenden NOx-Signal auch ein den Lambda-Wert darstellendes Lambda-Signal bzw. ein die Sauerstoffkonzentration darstellendes O₂-Signal ab und beide Signale sollen hinsichtlich ihres Dynamikverhaltens überprüft werden.

Bekannterweise kann die Brennkraftmaschine 1 so betrieben werden, daß der Lambda-Wert an der Vorkat-Lambda-Sonde 3 eine definierte Schwingung um Lambda = 1 ausführt. Nach dem Stand der Technik wird diese Schwingung dann dazu verwendet, das Dynamikverhalten der Vorkat-Lambda-Sonde 3 zu überprüfen. Dieses Vorgehen ist jedoch für den NOx-Meßaufnehmer 6 nicht geeignet, da dieser sich stromab des Drei-Wege-Vorkatalysators 4 und des NOx-Speicherkatalysators 5 befindet und die Schwingung um Lambda = 1 durch die beiden Katalysatoren deutlich gedämpft wird, so daß es nicht möglich ist, ein verlangsamtes Ansprechen bzw. ungenügendes Dynamikverhalten des NOx-Meßaufnehmers 6 zu erkennen.

In einer Regenerationsphase des NOx-Speicherkatalysators 5 ist es jedoch möglich, das Dynamikverhalten des NOx-Meßaufnehmers 6 zu überprüfen. In diesen Regenerationsphasen wird das im mageren Betrieb der Brennkraftmaschine 1 vom NOx-Speicherkatalysator 5 gespeicherte NOx umgesetzt. Zu dieser Regeneration wird von Lambda = 1 bzw. von Lambda > 1 auf ein definiert fettes Gemisch mit etwa Lambda = 0,8 geschaltet. Eine solche Regenerationsphase ist in Fig. 2 dargestellt. Die Kurve 7 zeigt den zeitlichen Verlauf des Lambda-Wertes des Rohabgases. Wie gut zu sehen ist, wird zu Beginn der Regenerationsphase zum Zeitpunkt t₁ von Lambda ≈ 1,4 auf Lambda = 0,8 geschaltet. Zu Ende der Regenerationsphase wird zum Zeitpunkt t₂ wieder der ursprüngliche Lambda-Wert von ca. 1,4 eingestellt. In Kurve 8 ist der zeitliche Verlauf des NOx-Signals eines voll funktionsfähigen NOx-Meßaufnehmers dargestellt. In der bzw. durch die Regenerationsphase wird kurzzeitig NOx freigesetzt. Dieses aus einer Desorption des NOx-Speicherkatalysator stammende NOx ist im NOx-Signal des NOx-Meßaufnehmers 6 an einem Desorptionspeak 9 gut zu erkennen. Gibt das NOx-Signal des NOx-Meßaufnehmers 6 eine oder beide Flanken dieses Desorptionspeaks 9 in einer Regenerationsphase nicht mit ausreichender Bandbreite, d.h. mit ungenügender Flankensteilheit wieder, muß ein fehlerhaftes Dynamikverhalten des NOx-Meßaufnehmers 6 bezüglich NOx diagnostiziert werden.

Gegen Ende der Regenerationsphase wird kurzzeitig auch Ammoniak (NH₃) gebildet. Da der NOx-Meßaufnehmer 6, wie er aus vorstehend zitierter Publikation bekannt ist, eine Querempfindlichkeit des NOx-Signals gegenüber NH₃ aufweist, wird gegen Ende der Regenerationsphase im NOx-Signal dieser NH₃-Peak 10 erfaßt. Wird eine oder beide Flanken des NH₃-Peaks 10 nicht mit ausreichender Flankensteilheit wiedergegeben, kann ebenfalls ein fehlerhaftes Dynamikverhalten des NOx-Meßaufnehmers 6 diagnostiziert werden.

Je nach verwendetem NOx-Speicherkatalysator 5 und Meßaufnehmer 6 können die Peaks 9 und 10 auch zusammenfallen.

Es sei darauf hingewiesen, daß der Desorptionspeak 9 und der NH₃-Peak 10 aufgrund von Reaktionen im NOx-Speicherkatalysator 5 selbst entstehen. Eine Generierung solcher Peaks durch Gemischänderungen im normalen Betrieb ist nahezu unmöglich, da beispielsweise von der Brennkraftmaschine 1 erzeugte NOx-Sprünge durch die Katalysatoren 4, 5 stark gedämpft werden.

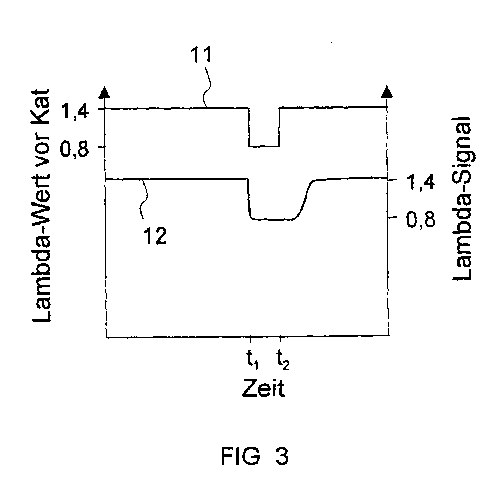

Liefert der NOx-Meßaufnehmer 6 zusätzlich noch ein Lambda-Signal bzw. ein O₂-Signal, kann dieses ebenfalls während der Regenerationsphase hinsichtlich des Dynamikverhaltens überprüft werden, wie in Fig. 3 dargestellt ist. Kurve 11 zeigt ähnlich der Kurve 7 der Fig. 2 den zeitlichen Verlauf des Lambda-Wertes des Rohabgases während einer Regenerationsphase. Kurve 12 gibt den zeitlichen Verlauf des Lambda-Signales eines voll funktionsfähigen NOx-Meßaufnehmers 6 wieder. Wird in der Regenerationsphase zum Zeitpunkt t₁ das Gemisch auf Lambda ≈ 0,8 geschaltet, kann an diesem Lambda-Sprung ins Fette die Dynamik des Lambda-Signales des NOx-Meßaufnehmers 6 überprüft werden. Wird ein solcher Mager-Fett-Sprung nicht mit ausreichender Bandbreite bzw. Flankensteilheit vom Lambda-Signal des NOx-Meßaufnehmers 6 wiedergegeben, kann ein fehlerhaftes Dynamikverhalten des Lambda-Signals des NOx-Meßaufnehmers 6 diagnostiziert werden. Natürlich kann auch der Fett-Mager-Sprung am Ende der Regenerationsphase zur Diagnose verwendet werden.

Das Ausnutzen eines Sprunges des Lambda-Wertes des Rohabgases bei Beginn einer Regenerationsphase hat den Vorteil, daß ein solcher Lambda-Sprung eine deutlich größere Amplitude aufweist als eine im normalen Betrieb auftretende Schwingung des Lambda-Wertes stromauf der Katalysatoren 4, 5, die ja zudem noch durch die Katalysatoren 4, 5 stark gedämpft wird. Somit steht in der Regenerationsphase am Einbauort des NOx-Meßaufnehmers 6 stromab der Katalysatoren 4 und 5 eine starke Änderung des Lambda-Wertes zur Verfügung, die zur Überprüfung des Dynamikverhaltens des Lambda-Signales bzw. des O₂-Signales des NOx-Meßaufnehmers 6 ausgenutzt werden kann.

## Patentansprüche

1. Verfahren zur Überprüfung des Dynamikverhaltens eines Messaufnehmers (6), der mindestens eine Stoffkonzentration im Abgas einer bei normalem Betrieb mit Luftüberschuss arbeitenden Brennkraftmaschine (1) stromab eines NOx-Speicherkatalysators (5) erfasst, welcher in einer Regenerationsphase unter Zugabe eines Reduktionsmittels gespeichertes NOx katalytisch umsetzt, wobei das Reduktionsmittel durch kurzzeitigen Betrieb der Brennkraftmaschine (1) mit einem fetten Luft/Kraftstoffgemisch erzeugt wird, **dadurch gekennzeichnet, daß** während der Regenerationsphase eine Änderungsrate mindestens einer Stoffkonzentration überwacht wird und ein fehlerhaftes Dynamikverhalten des diese Stoffkonzentration darstellenden Signals des Meßaufnehmers (6) diagnostiziert wird, wenn die Änderungsrate vom Signal des Meßaufnehmers (6) nicht mit für ein korrektes Dynamikverhalten des Messaufnehmers ausreichender Flankensteilheit wiedergegeben wird.

2. Verfahren nach Anspruch 1, **dadurch gekennzeichnet, daß** während der Regenerationsphase eine in Form eines NOx-Desorptionspeaks (9) auftretende Stoffkonzentrationsänderung überwacht wird und bei einem Messaufnehmer (6), der ein die NOx-Konzentration im Abgas darstellendes NOx-Signal (8) abgibt, das NOx-Signal (8) dahingehend überwacht wird, ob mindestens eine der Flanken des NOx-Desorptionspeaks (9) mit für ein korrektes Dynamikverhalten des Messaufnehmers ausreichender Flankensteilheit wiedergegeben wird.

3. Verfahren nach einem der vorhergehenden Ansprüche, **dadurch gekennzeichnet, daß** gegen Ende der Regenerationsphase eine in Form eines NH3-Peaks (10) auftretende Stoffkonzentrationsänderung überwacht wird und bei einem Messaufnehmer (6), der ein die NOx-Konzentration im Abgas darstellendes NOx-Signal (8) abgibt und eine Querempfindlichkeit gegen NH₃ aufweist, das NOx-(8) Signal dahingehend überwacht wird, ob mindestens eine der Flanken des NH₃-Peaks (10) mit für ein korrektes Dynamikverhalten des Messaufnehmers ausreichender Flankensteilheit wiedergegeben wird.

4. Verfahren nach Anspruch 2 und 3, **dadurch gekennzeichnet, daß** bei der überwachten Stoffkonzentrationsänderung NOx-Desorptionspeak (9) und NH₃-Peak (10) zusammenfallen.

5. Verfahren nach einem der vorhergehenden Ansprüche, **dadurch gekennzeichnet, daß** zu Beginn der Regenerationsphase eine in Form eines Sprungs des Lambda-Wertes von magerem zu fettem Gemisch auftretende Stoffkonzentrationsänderung überwacht wird und bei einem Messaufnehmer (6), der ein den Lambda-Wert im Abgas darstellendes Lambda-Signal (12) abgibt, das Lambda-Signal (12) dahingehend überwacht wird, ob der Sprung mit für ein korrektes Dynamikverhalten des Messaufnehmers ausreichender Flankensteilheit wiedergegeben wird.

6. Verfahren nach einem der vorhergehenden Ansprüche, **dadurch gekennzeichnet, daß** am Ende der Regenerationsphase eine in Form eines Sprungs des Lambda-Wertes von fettem zu magerem Gemisch auftretende Stoffkonzentrationsänderung überwacht wird und bei einem Messaufnehmer (6), der ein den Lambda-Wert im Abgas darstellendes Lambda-Signal (12) abgibt, das Lambda-Signal (12) dahingehend überwacht wird, ob der Sprung mit für ein korrektes Dynamikverhalten des Messaufnehmers ausreichender Flankensteilheit wiedergegeben wird.

7. Verfahren nach einem der vorhergehenden Ansprüche, **dadurch gekennzeichnet, daß** zu Beginn der Regenerationsphase eine in Form eines Sprungs der Sauerstoffkonzentration von magerem zu fettem Gemisch auftretende Stoffkonzentrationsänderung überwacht wird und bei einem Messaufnehmer (6), der ein die Sauerstoffkonzentration im Abgas darstellendes O₂-Signal abgibt, das O₂-Signal dahingehend überwacht wird, ob der Sprung mit für ein korrektes Dynamikverhalten des Messaufnehmers ausreichender Flankensteilheit wiedergegeben wird.

8. Verfahren nach einem der vorhergehenden Ansprüche, **dadurch gekennzeichnet, daß** am Ende der Regenerationsphase eine in Form eines Sprungs der Sauerstoffkonzentration von fettem zu magerem Gemisch auftretende Stoffkonzentrationsänderung überwacht wird und bei einem Messaufnehmer (6), der ein die Sauerstoffkonzentration im Abgas darstellendes O₂-Signal abgibt, das O₂-Signal dahingehend überwacht wird, ob der Sprung mit für ein korrektes Dynamikverhalten des Messaufnehmers ausreichender Flankensteilheit wiedergegeben wird.

## Claims

1. Method for checking the dynamic behaviour of a measuring sensor (6) which detects at least one substance concentration in the exhaust gas of an internal combustion engine (1) operating in normal operation with air excess, downstream of an NOx storage catalyst (5) which, in a regeneration phase, catalytically converts stored NOx by the addition of a reducing agent, the reducing agent being produced by the internal combustion engine (1) being operated briefly with a rich air/fuel mixture, **characterized in that**, during a regeneration phase, a rate of change of at least one substance concentration is monitored and a faulty dynamic behaviour of the signal representing this substance concentration and coming from the measuring sensor (6) is diagnosed when the rate of change is not reproduced with sufficient edge steepness for correct dynamic behaviour of the measuring sensor by the signal from the measuring sensor (6).

2. Method according to Claim 1, **characterized in that**, during the regeneration phase, a substance concentration change occurring in the form of an NOx desorption peak (9) is monitored, and, in the case of a measuring sensor (6) emitting an NOx signal (8) representing the NOx concentration in the exhaust gas, the NOx signal (8) is monitored as to whether at least one of the edges of the NOx desorption peak (9) is reproduced with sufficient edge steepness for correct dynamic behaviour of the measuring sensor.

3. Method according to one of the preceding claims, **characterized in that**, towards the end of the regeneration phase, a substance concentration change occurring in the form of an NH₃ peak (10) is monitored, and, in the case of a measuring sensor (6) which emits an NOx signal (8) representing the NOx concentration in the exhaust gas and exhibits cross-sensitivity to NH₃, the NOx signal (8) is monitored as to whether at least one of the edges of the NH₃ peak (10) is reproduced with sufficient edge steepness for correct dynamic behaviour of the measuring sensor.

4. Method according to Claims 2 and 3,
**characterized in that**, during the monitored substance concentration change, the NOx desorption peak (9) and the NH₃ peak (10) coincide.

5. Method according to one of the preceding claims, **characterized in that**, at the start of the regeneration phase, a substance concentration change occurring in the form of a jump of the lambda value from a lean to a rich mixture is monitored, and, in the case of a measuring sensor (6) emitting a lambda signal (12) representing the lambda value in the exhaust gas, the lambda signal (12) is monitored as to whether the jump is reproduced with sufficient edge steepness for correct dynamic behaviour of the measuring sensor.

6. Method according to one of the preceding claims, **characterized in that**, at the end of the regeneration phase, a substance concentration change occurring in the form of a jump of the lambda value from a rich to a lean mixture is monitored, and, in the case of a measuring sensor (6) emitting a lambda signal (12) representing the lambda value in the exhaust gas, the lambda signal (12) is monitored as to whether the jump is reproduced with sufficient edge steepness for correct dynamic behaviour of the measuring sensor.

7. Method according to one of the preceding claims, **characterized in that**, at the start of the regeneration phase, a substance concentration change occurring in the form of a jump of the oxygen concentration from a lean to a rich mixture is monitored, and, in the case of a measuring sensor (6) emitting an O₂ signal representing the oxygen concentration in the exhaust gas, the O₂ signal is monitored as to whether the jump is reproduced with sufficient edge steepness for correct dynamic behaviour of the measuring sensor.

8. Method according to one of the preceding claims, **characterized in that**, at the end of the regeneration phase, a substance concentration change occurring in the form of a jump of the oxygen concentration from a rich to a lean mixture is monitored, and, in the case of a measuring sensor (6) emitting an O₂ signal representing the oxygen concentration in the exhaust gas, the O₂ signal is monitored as to whether the jump is reproduced with sufficient edge steepness for correct dynamic behaviour of the measuring sensor.

## Revendications

1. Procédé de contrôle du comportement dynamique d'un capteur de mesure (6) qui détecte, en aval d'un catalyseur de stockage de NOx (5), au moins une concentration de matière dans le gaz d'échappement provenant d'un moteur à combustion interne (1) fonctionnant, en régime normal, en excès d'air, catalyseur qui, dans une étape de régénération, procède, par l'ajout d'un réducteur, à une réaction catalytique du NOx stocké, le réducteur étant généré au moyen d'un fonctionnement momentané du moteur à combustion interne (1) avec un mélange air/carburant riche, **caractérisé en ce que** l'on supervise, pendant l'étape de régénération, le taux de variation d'au moins une concentration de matière, et, **en ce que** l'on déduit un comportement dynamique défectueux du signal du capteur de mesure (6) devant indiquer la concentration de matière en question, dans le cas où le signal du capteur de mesure (6) ne transmet pas le taux de variation avec une pente du signal suffisante pour un comportement dynamique correct du capteur de mesure.

2. Procédé selon la revendication 1, **caractérisé en ce que** l'on supervise, pendant l'étape de régénération, une variation de la concentration de matière représentée par un pic de désorption de NOx (9), et **en ce que** l'on supervise le signal de NOx (8) d'un capteur de mesure (6) qui émet un signal NOx (8) représentant la concentration du NOx dans le gaz d'échappement, en procédant de sorte que l'on vérifie si au moins une des pentes du pic de désorption de NOx (9) est transmise avec une pente du signal suffisante pour un comportement dynamique correct du capteur de mesure.

3. Procédé selon une quelconque des revendications précédentes, **caractérisé en ce que** l'on supervise, vers la fin de l'étape de régénération, une variation de la concentration de matière représentée par un pic de NH₃ (10), et **en ce que** l'on supervise le signal de NOx (8) d'un capteur de mesure (6) qui émet un signal NOx (8) représentant la concentration du NOx dans le gaz d'échappement et qui présente une sensibilité transverse au NH₃, en procédant de sorte que l'on vérifie si au moins une des pentes du pic de désorption de NH₃ (10) est transmise avec une pente du signal suffisante pour un comportement dynamique correct du capteur de mesure.

4. Procédé selon les revendications 2 et 3, **caractérisé en ce que**, lors de la variation supervisée de la concentration de matière, le pic de désorption de NOx (9) et le pic de NH₃ (10) coïncident.

5. Procédé selon une quelconque des revendications précédentes, **caractérisé en ce que** l'on supervise, au début de l'étape de régénération, une variation de la concentration de matière, en partant d'un mélange pauvre vers un mélange riche, variation qui est représentée par un saut de la valeur lambda, **en ce que** l'on supervise le signal lambda (12) d'un capteur de mesure (6) qui émet un signal lambda (12) représentant la valeur lambda dans le gaz d'échappement, en procédant de sorte que l'on vérifie que le saut est transmis avec une pente du signal suffisante pour un comportement dynamique correct du capteur de mesure.

6. Procédé selon une quelconque des revendications précédentes, **caractérisé en ce que** l'on supervise, à la fin de l'étape de régénération, une variation de la concentration de matière, en partant d'un mélange riche vers un mélange pauvre, variation qui est représentée par un saut de la valeur lambda, et **caractérisé en ce que** l'on supervise le signal lambda (12) d'un capteur de mesure (6) qui émet un signal lambda (12) représentant la valeur lambda dans le gaz d'échappement, en procédant de sorte que l'on vérifie que le saut est transmis avec une pente du signal suffisante pour un comportement dynamique correct du capteur de mesure.

7. Procédé selon une quelconque des revendications précédentes, **caractérisé en ce que** l'on supervise, au début de l'étape de régénération, une variation de la concentration de matière, en partant d'un mélange pauvre vers un mélange riche, variation qui est représentée par un saut de la concentration en oxygène, et **en ce que** l'on supervise le signal d'O₂ d'un capteur de mesure (6) qui émet un signal d'O₂ représentant la concentration en oxygène dans le gaz d'échappement, en procédant de sorte que l'on vérifie que le saut est transmis avec une pente du signal suffisante pour un comportement dynamique correct du capteur de mesure.

8. Procédé selon une quelconque des revendications précédentes, **caractérisé en ce que** l'on supervise, à la fin de l'étape de régénération, une variation de la concentration de matière, en partant d'un mélange riche vers un mélange pauvre, variation qui est représentée par un saut de la concentration en oxygène, et **caractérisé en ce que** l'on supervise le signal d'O₂ d'un capteur de mesure (6) qui émet un signal d'O₂ représentant la concentration en oxygène dans le gaz d'échappement, en procédant de sorte que l'on vérifie que le saut est transmis avec une pente du signal suffisante pour un comportement dynamique correct du capteur de mesure.
